# EUROPÄISCHE PATENTSCHRIFT

(11) **EP 1 920 727 B1**
(45) Veröffentlichungstag und Bekanntmachung des Hinweises auf die Patenterteilung: **20.01.2010**
(21) Anmeldenummer: 06090206.1
(22) Anmeldetag: 09.11.2006
(51) Int. Cl.: A61B 19/00, A61B 17/34

(54) **Verdrehdichtung**
Torsion seal
Joint hermétique torsadé

(43) Veröffentlichungstag der Anmeldung: 14.05.2008
(73) Patentinhaber: BANDELIN electronic GmbH & Co. KG, 12207 Berlin (DE)
(72) Erfinder: Dirk Gebauer, 12249 Berlin (DE); Rainer Jung, 13125 Berlin (DE); Sebastian Kähler, 13158 Berlin (DE)
(74) Vertreter: Pfenning, Meinig & Partner GbR

(56) Entgegenhaltungen:
- WO-A1-2005/058409
- DE-U1- 8 413 820
- DE-U1- 29 821 739
- FR-A1- 2 863 504
- US-A1- 5 957 913
- US-A1- 2005 155 611
- US-B1- 6 589 167

## Beschreibung

Die Erfindung betrifft eine Verdrehdichtung, ein Gefäß mit einer erfindungsgemäßen Verdrehdichtung als Deckel, sowie eine Verwendung des Gefäßes.

Es ist bekannt, im Bereich der minimalinvasiven Chirurgie (MIC) Rohrschaftinstrumente oder Hohlinstrumente zu verwenden. Um Rohrschaftinstrumente nach Gebrauch zu Reinigen ist es bekannt, durch den Rohrschaft bzw. Hohlraum hindurch eine Flüssigkeit zu pumpen, siehe beispielsweise die Druckschrift DE 44 41 401 A1.

DE 29821739 offenbart eine Vorrichtung gemäss der Oberbegriff des Anspruchs 1.

Um zu gewährleisten, dass Flüssigkeit durch die Rohrschafte, die in der Regel einen sehr kleinen Durchmesser aufweisen, gepumpt wird, ist es notwendig, das Ende, an dem gepumpt werden soll, gegen das Ende, an dem Flüssigkeit in den Rohrschaft gesaugt werden soll, gut gegeneinander abzudichten.

Des Weiteren können die Rohrschaftinstrumente unterschiedliche Außendurchmesser aufweisen. Es ist deswegen vorteilhaft, wenn eine Dichtung diese unterschiedlichen Außendurchmesser berücksichtigt.

Die Druckschrift DE 82 11 691 U1 offenbart eine Dichtungsmanschette aus elastischem Material für Wanddurchführungen, wobei die Manschette konisch ausgebildet und mit unterschiedlich großen Öffnungsquerschnitten versehen ist. Durch diese können Gegenstände abgedichtet werden, deren Außendurchmesser im Zwischenbereich der Durchmesser der beiden Öffnungsquerschnitte der Dichtungsmanschette liegen. Nachteil ist allerdings, dass die Dichtwirkung der Dichtungsmanschette abhängig ist von dem Außendurchmesser des durchzuführenden Objekts, insbesondere eine gute Abdichtung von Objekten mit Außendurchmessern im Bereich des kleineren der beiden Öffnungsquerschnitte der Dichtungsmanschette unzureichend sein kann, und eine Dichtung von Gegenständen mit einem Außendurchmesser kleiner als der kleinere der beiden Öffnungsquerschnitte der Dichtungsmanschette überhaupt nicht möglich ist.

Die Druckschrift DE 103 33 365 A1 offenbart ebenfalls eine Dichtung mit variablem Innendurchmesser. Die Dichtung weist mindestens eine ringförmige Dichtungsscheibe aus einem zumindest flexiblen und/oder elastischen Material mit einer an die Querschnittsform des durchzuführenden Objektes angepassten Öffnung auf, wobei die Dichtungsscheibe in Umfangsrichtung der Öffnung ein wellenförmiges Profil besitzt und die Öffnung der Dichtungsscheibe eine an die Mantelfläche des durchzuführenden Elements anlegbare und eine geschlossene Abdichtfläche auf der Mantelfläche des durchzuführenden Elements bildende innere Stirnfläche hat. Der Öffnungsquerschnitt kann in diesem Fall durch eine Streckung oder Steifung des wellenförmigen Profils der Dichtungsscheibe erfolgen. Nachteil ist, dass eine Veränderung des Öffnungsquerschnitts unter Beibehaltung einer ausreichenden Dichtwirkung nur über einen begrenzten Bereich möglich ist.

Aufgabe der Erfindung ist es somit, eine Dichtung mit einer in ihrem Öffnungsquerschnitt variierbaren Öffnung zu schaffen, die eine Änderung des Öffnungsquerschnitts in einem sehr weiten Bereich ermöglicht und gleichzeitig eine hohe Dichtwirkung gewährleisten kann, leicht zu bedienen, sowie kostengünstig herstellbar ist. Eine weitere Aufgabe der Erfindung ist es, ein Gefäß zu schaffen, welches eine derartige Dichtung aufweist, sowie eine vorteilhafte Verwendung eines derartigen Gefäßes vorzuschlagen.

Diese Aufgaben werden durch eine Dichtung, ein Gefäß und eine Verwendung des Gefäßes nach den unabhängigen Ansprüchen gelöst.

Vorteilhafte Weiterbildungen werden in den abhängigen Ansprüchen beschrieben.

Die Erfindung schafft eine Verdrehdichtung, enthaltend ein Dichtelement und ein Haltemittel, wobei das Dichtelement als flächiges Gebilde mit einer in ihrem Öffnungsquerschnitt veränderbaren Öffnung ausgebildet ist und das Haltemittel ein erstes Halteteil und ein zweites Halteteil aufweist, die gegeneinander verdrehbar sind, und erstes Halteteil und zweites Halteteil jeweils zumindest an einer Befestigungsstelle mit dem Dichtelement fest verbunden sind, wobei die Befestigungsstellen derart gewählt sind, dass durch ein Verdrehen des ersten und zweiten Halteteils das Dichtelement derart verformbar ist, dass sich die Öffnung des Dichtelements in ihrem Öffnungsquerschnitt reduziert.

Die Halteteile können beispielsweise manuell, motorisch, pneumatisch oder hydraulisch gegeneinander verdrehbar ausgebildet sein.

Im Folgenden soll der Einfachheit halber nur von "Befestigungsstellen" gesprochen werden, was aber auch, wenn nicht ausdrücklich anders gesagt, nur eine einzige Befestigungsstelle für erstes und/oder zweites Halteteil einschließen soll.

Erfindungsgemäß schnürt sich durch Drehen der Halteteile das an den Halteteilen befestigte Dichtelement mehr und mehr zusammen, wodurch sich der Öffnungsquerschnitt der Öffnung des Dichtelements verkleinert. Befindet sich ein Objekt in der Öffnung, beispielsweise ein Rohrschaftinstrument, so legt sich das Dichtelement beim Verdrehen der Halteteile zunächst an die Außenfläche des Schaftes an, und wird beim weiteren Verdrehen der Halteteile stärker und stärker gegen diese Außenfläche angepresst. Auf diese Weise lässt sich nicht nur der zunächst offene Zwischenraum zwischen Schaft und Dichtelement schließen, sondern auch eine hohe Dichtwirkung erzielen.

Dadurch, dass sich das Dichtelement beim Verdrehen der Halteteile zusammenschnürt, kann der Öffnungsquerschnitt der Öffnung des Dichtelements über einen großen Bereich variiert werden, insbesondere ist es bei geeigneter Ausbildung des Dichtelements möglich, die Öffnung des Dichtelements vollständig zu verschließen.

Dadurch, dass nur ein Verdrehen der Halteteile notwendig ist, um die Öffnung des Dichtelementes zu reduzieren oder zu verschließen, ist die Verdrehdichtung besonders einfach bedienbar.

Vorzugsweise ist das Dichtelement elastisch verformbar, besteht somit bereichsweise, vorzugsweise vollständig, aus einem elastischen Material.

Durch die Verwendung eines elastischen Materials kann nicht nur gewährleistet werden, dass das Dichtelement genügend verformbar ist, um den Öffnungsquerschnitt über einen großen Bereich reduzieren, wenn nicht sogar vollständig die Öffnung verschließen zu können, sondern kann auch gewährleisten, dass das Dichtelement beim Zurückdrehen der Halteteile in seine Ursprungsform zurückkehrt. Dies ist insbesondere dann sinnvoll, wenn die Dichtung mehrfach verwendet werden soll und die Öffnung des Dichtelements im unverformten Zustand dieselbe Form aufweisen soll.

Das oder die Materialien des Dichtelements besitzen besondere physikalische Parameter, um eine hohe mechanische Beanspruchung beim Verdrehen über einen weiten Durchmesserbereich mit hoher Lastwechselzahl (ca. 500 Benutzungen ohne Ausfall) gewährleisten zu können. Vorteilhafterweise ist sie gegenüber chemischen Desinfektionsmitteln beständig, enthält keine Weichmacher und kann durch Ultraschall nicht beschädigt werden.

Vorteilhafterweise weist diesbezlüglich der elastische Bereich des Dichtelements eine Reißdehnung von mindestens 300%, vorzugsweise mindestens 600%, besonders bevorzugt mindestens 800% auf. Vorzugsweise liegt die Shore-A-Härte im Bereich von 20 - 40. Der Weiterreißwiderstand liegt vorzugsweise im Bereich von 10 - 30 N/mm.

Vorzugsweise besteht das Dichtelement zumindest bereichsweise, besonders bevorzugt vollständig, aus Silikonkautschuk, insbesondere additionsvernetzten Silikonkautschuktypen, thermoplastischen Elastomeren, insbesondere "Mediprene", Latex (Naturkautschuk) und/oder Polymerlatex.

Vorzugsweise besteht das Dichtelement aus einem spritzgussfähigen Material bzw. aus spritzgussfähigen Materialien.

Vorzugsweise ist die Fläche des Dichtelements geschlossen.

Eine erfindungsgemäße Verdrehdichtung kann insbesondere für die Abdichtung gegen Fluide, worunter neben gasförmigen und flüssigen insbesondere auch pastöse Medien verstanden werden sollen, eingesetzt werden.

Eine vorteilhafte Weiterbildung der Erfindung sieht vor, dass zumindest erstes und/oder zweites Halteteil mit dem Dichtelement ununterbrochen entlang einer auf der Fläche des Dichtelements die Öffnung des Dichtelements umlaufenden, geschlossenen Linie befestigt ist bzw. sind.

Durch eine derartige umlaufende Befestigung kann eine hohe Dichtwirkung zwischen Dichtelement und Haltemittel erreicht werden. Vorzugsweise sind beide Haltemittel auf diese Weise an dem Dichtelement befestigt.

Bevorzugt ist, insbesondere wenn das Dichtelement aus einem elastischen Material besteht, das Dichtelement kraftschlüssig mit dem ersten und/oder zweiten Halteteil verbunden, beispielsweise durch Einklemmen.

Grundsätzlich ist auch eine stoffschlüssige oder formschlüssige Verbindung nicht ausgeschlossen, wobei für letztere das Dichtelement vorzugsweise einen starren Bereich aufweist, damit der Formschluss gehalten werden kann. Eine Kombination der verschiedenen Befestigungsmöglichkeiten ist selbstverständlich ebenfalls möglich.

Eine weitere vorteilhafte Weiterbildung der Erfindung sieht vor, dass das erste Halteteil gegen das zweite Halteteil um eine Drehachse verdrehbar ist, wobei die Befestigungsstellen von erstem und zweitem Halteteil an dem Dichtelement derart gewählt sind, dass es keine senkrecht zur Drehachse orientierte Ebene gibt, auf der sich sowohl eine Befestigungsstelle des ersten als auch eine Befestigungsstelle des zweiten Halteteils befinden.

Dies hat zur Folge, dass es parallel zur Drehachse immer einen Abstand zwischen einer ersten und zweiten Befestigungsstelle gibt. Dieser Abstand ermöglicht ein definiertes Zusammenschnüren des Dichtelements.

Je nach Ausbildung des Dichtelements und Verteilung der Befestigungsstellen ist es möglich, dass sich das Dichtelement an mehreren Stellen zusammenschnürt.

Eine weitere vorteilhafte Weiterbildung der Erfindung sieht vor, dass das Dichtelement in Form einer Scheibe, eines Rings oder eines Schlauchs ausgebildet ist.

Eine gewisse axiale Ausdehnung des Dichtelements ist grundsätzlich vorteilhaft, damit sich das Dichtelement, bei geeigneter Wahl der Befestigungsstellen, aufgrund der Drehung definiert zusammenschnüren kann.

Eine derartige axiale Ausdehnung ist jedoch für die Gewährleistung der Funktion der Dichtung nicht zwingend notwendig. Insbesondere können auch sämtliche Befestigungsstellen des ersten und zweiten Halteteils in einer gemeinsamen Ebene senkrecht zur Drehachse der Halteteile liegen.

Eine weitere vorteilhafte Weiterbildung der Erfindung sieht vor, dass das Dichtelement zumindest in einem Teilbereich umlaufend der Öffnung nach innen gewölbt ist, und sich die Befestigungsstellen des ersten Halteteils in dem einen und die Befestigungsstellen des zweiten Halteteils in dem anderen der durch den Scheitel der Wölbung voneinander getrennten Bereiche des Dichtelements befinden.

Bevorzugt wird durch den nach innen gewölbten Bereich festgelegt, wo sich das Dichtelement beim Verdrehen der Halteteile zusammenschnürt.

Grundsätzlich ist auch eine Wölbung nach außen möglich, erfordert dann aber mehr Drehungen zum Reduzieren des Öffnungsquerschnitts der Öffnung des Dichtelements. Auch eine mehrfache Wölbung, also ein gewelltes Profil des Dichtelements ist möglich.

Eine weitere vorteilhafte Weiterbildung der Erfindung sieht vor, dass das Dichtelement rotationssymmetrisch ausgebildet ist, und die Befestigungsstellen des ersten Halteteils und des zweiten Halteteils an dem Dichtelement auf jeweils einem senkrecht zur Symmetrieachse des Dichtelements bei häufigem Einsatz orientierten ersten bzw. zweiten Kreis liegen.

Durch die rotationssymmetrische Ausbildung des Dichtelements und der kreisförmigen Anordnung der Befestigungsstellen um die Symmetrieachse des Dichtelements wird eine möglichst gleichmäßige mechanische Belastung des Dichtelements beim Verdrehen der Halteteile erreicht. Dies ist insbesondere für die Haltbarkeit des Dichtelements entscheidend.

Eine weitere vorteilhafte Weiterbildung der Erfindung sieht vor, dass der Radius des ersten Kreises mit dem zweiten Kreis identisch ist.

Diese Symmetrie ist vorteilhaft für die Verringerung der Belastung des Dichtelements beim Verdrehen der Halteteile. Grundsätzlich sind aber auch unterschiedliche Radien möglich, also eine asymmetrische Anordnung der Befestigungsstellen.

Eine weitere vorteilhafte Weiterbildung der Erfindung sieht vor, dass das erste Halteteil gegen das zweite Halteteil um genau eine Drehachse verdrehbar ist.

Vorzugsweise ist diese Drehachse die Symmetrieachse des Dichtelements.

Eine weitere vorteilhafte Weiterbildung der Erfindung sieht vor, dass das Haltemittel ein Arretiermittel aufweist, mit dem erstes Halteteil und zweites Halteteil in einer zueinander verdrehten Stellung arretierbar sind.

Dies ist insbesondere dann vorteilhaft, wenn das Dichtelement elastisch ist, also eine Rückstellkraft auf die gegeneinander verdrehten Halteteile aufgrund des verformten Dichtelements wirkt.

Ein derartiges Arretiermittel kann konstruktiv beispielsweise über eine Rastverbindung realisiert werden, bei der beispielsweise Vorsprünge des ersten Halteteils in jeweils eine Nut des anderen Halteteils formschlüssig eingreifen. Die Nuten können einseitig angeschrägt sein, um ein einfaches Weiterdrehen der Halteteile in einer Drehrichtung zu ermöglichen. Die beiden Halteteile können in ihrer Grundposition zurückgestellt werden, indem zunächst das Halteteil mit den Vorsprüngen vom anderen Halteteil weggezogen wird, so dass die Vorsprünge die Aussparungen verlassen, und dann zurückgedreht wird.

Eine weitere vorteilhafte Weiterbildung der Erfindung sieht vor, dass das erste und/oder das zweite Halteteil eine Öffnung aufweist bzw. aufweisen, die fluchtend mit der Öffnung des Dichtelements angeordnet ist bzw. sind.

Eine weitere vorteilhafte Weiterbildung der Erfindung sieht vor, dass die Öffnungsquerschnitte der Öffnungen des ersten und zweiten Halteteils in einem Grundzustand der Halteteile in Fluchtlinie vollständig den Öffnungsquerschnitt des Dichtelements überdecken.

Vorzugsweise weisen beiden Halteteile eine derartige Öffnung auf. Durch solche Öffnungen kann die Gefahr vermindert werden, dass Objekte, die durch die Öffnung des Dichtelements hindurchgeführt werden sollen, beim Durchführen direkt gegen das Dichtelement stoßen und das Dichtelement beschädigen.

Des Weiteren schafft die Erfindung ein Gefäß mit einem Deckel und einem Behälter, bei dem der Deckel durch eine Verdrehdichtung gemäß einem der Ansprüche 1 bis 14 gebildet wird, wobei ein Halteteil der Verdrehdichtung fluiddicht an dem Behälter befestigt ist, und wobei das Gefäß einen zum Innenvolumen des Behälters hin durchgeführten Anschluss für eine Pumpe zum Pumpen des Innenvolumens des Behälters aufweist.

Insbesondere kann ein Teil eines Halteteils oder das gesamte Halteteil Teil des Gefäßes sein, beispielsweise einstückig mit dem Gefäß ausgebildet sein.

Ein derartiges Gefäß eignet sich insbesondere für die Spülung von hohlen, rohrförmigen Elementen, insbesondere von medizinischen Hohlinstrumenten bzw. Rohrschaftinstrumenten.

Insbesondere die erfindungsgemäße Verdrehdichtung eignet sich selbstverständlich auch für andere Zwecke.

Als weitere Anwendung kann die erfindungsgemäße Verdrehdichtung beispielsweise als Durchführung zwischen zwei Räumen fungieren, beispielsweise als Durchführung für Kabel. Ein Halteteil kann in diesem Fall als Teil einer Wand ausgebildet sein. Des Weiteren lässt sich die erfindungsgemäße Verdrehdichtung für schwingungsgedämpfte Rohr- und Leitungsdurchführungen verwenden. Eine weitere Anwendung ist die Ausbildung der Verdrehdichtung als Schnellverschluss von Trinkflaschen oder medizinischen Versorgungsflaschen. Eine weitere Anwendung der Verdrehdichtung ist die Verwendung der Verdrehdichtung für schnell zu betätigende Kabel- und Rohreinführungen einschließlich für Steckverbindungsteile für militärische ABC-Container oder mobile Baustelleneinrichtungen.

Die Erfindung wird nun anhand von Ausführungsbeispielen, welche durch mehrere Figuren dargestellt sind, näher erläutert. Dabei zeigt:
- Fig. 1: ein Gefäß mit einer erfindungsgemäßen Verdrehdichtung,
- Fig. 2: das Gefäß in einer Explosionsdarstellung,
- Fig. 3a: einen Schnitt des in der Verdrehdichtung verwendeten Dichtelements,
- Fig. 3b: eine perspektivische Darstellung dieses Dichtelements,
- Fig. 4: eine Prinzipskizze des Dichtelements mit einem in die Öffnung des Dichtelements eingeführten rohrförmigen Element,
- Fig. 5: eine zweite Ausführungsform eines erfindungsgemäßen Dichtelements,
- Fig. 6a und Fig. 6b: eine dritte Ausführungsform eines erfindungsgemäßen Dichtelements, und
- Fig. 7: eine vierte Ausführungsform eines erfindungsgemäßen Dichtelements.

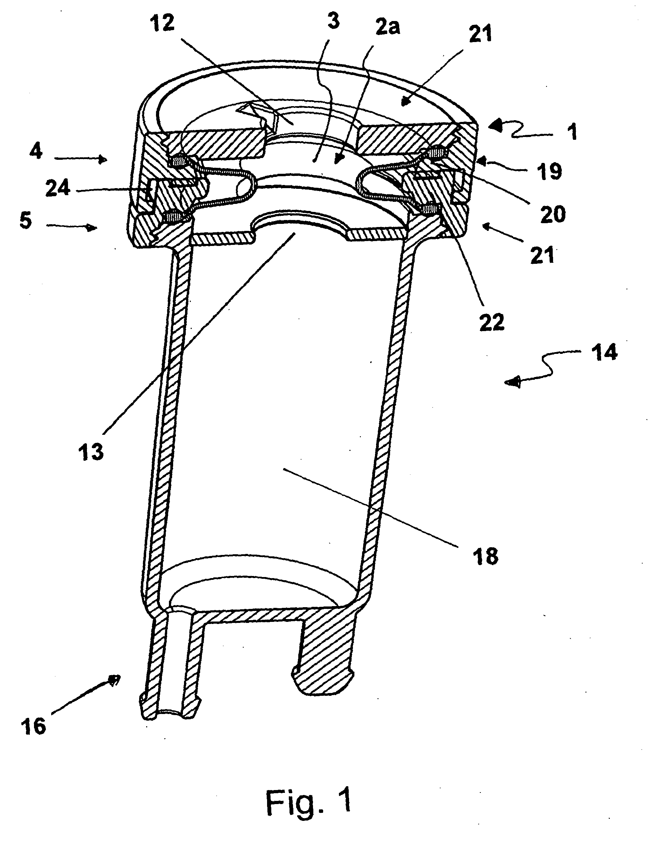

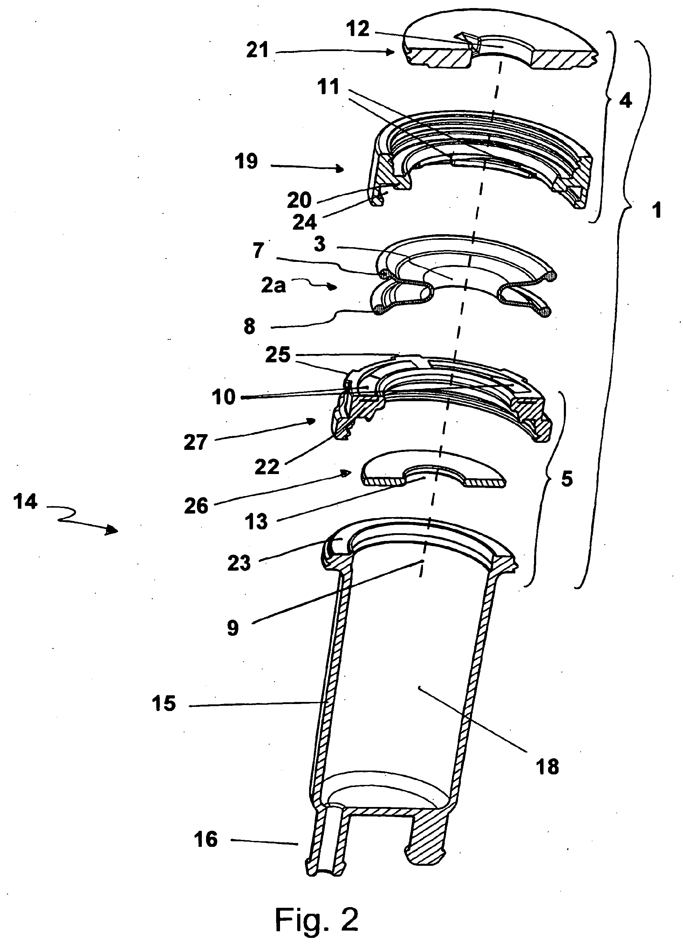

Die Fig. 1 zeigt ein Gefäß 14 mit einem Deckel und einem Behälter 15, bei dem der Deckel durch eine Ausführungsform einer erfindungsgemäßen Verdrehdichtung 1 gebildet wird. Der Übersicht wegen ist die vordere Hälfte des Gefäßes weggelassen. Die weggelassene andere Hälfte ergibt sich im Wesentlichen durch die Spiegelung der gezeigten Hälfte. Fig. 2 zeigt dasselbe Gefäß 14 in einer Explosionsdarstellung.

Die Verdrehdichtung 1 enthält ein Dichtelement 2a und ein Haltemittel. Das Dichtelement 2a ist als flächiges Gebilde mit einer in ihrem Öffnungsquerschnitt veränderbaren Öffnung 3 ausgebildet ist. Das Haltemittel weist ein erstes Halteteil 4 und ein zweites Halteteil 5 auf, die gegeneinander verdrehbar sind. Erstes Halteteil 4 und zweites Halteteil 5 sind jeweils an Befestigungsstellen 7, 8 fest mit dem Dichtelement 2a verbunden.

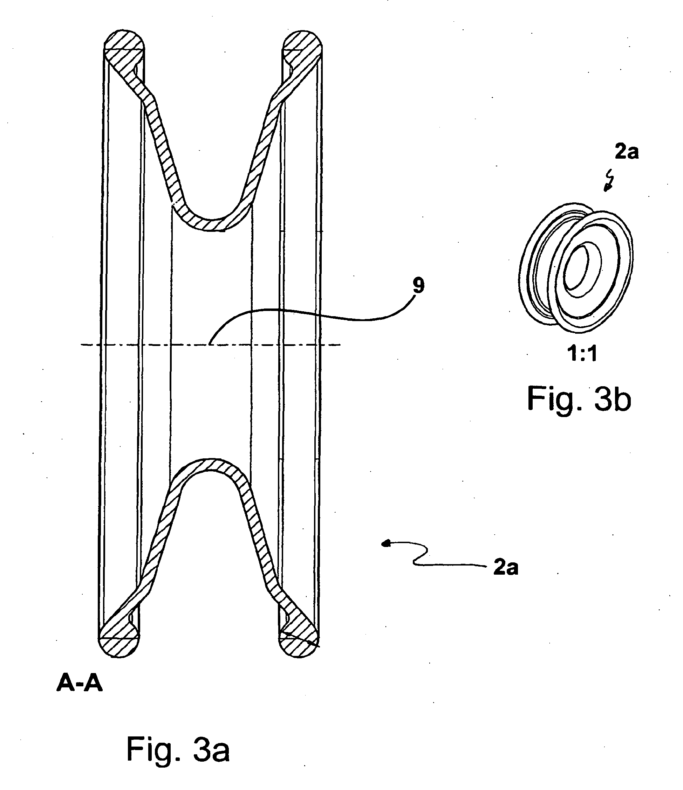

In diesem Ausführungsbeispiel ist das Dichtelement 2a als Ring mit rotationssymmetrischer Form ausgebildet. (siehe auch Fign. 3a und 3b) Abgesehen von der Öffnung 3, welche sich im Zentrum des Dichtelements 2a befindet, ist die Fläche des Dichtelements 2a geschlossen.

Das Dichtelement 2a ist umlaufend der Öffnung 3 nach innen gewölbt. Bezüglich der Ebene, in der der Scheitel der Wölbung liegt, die also senkrecht zur Symmetrieachse 9 ist, ist das Dichtelement 2a spiegelsymmetrisch ausgebildet.

An seinen jeweils oben und unten liegenden Außenrändern ist das ringförmige Dichtelement 2a zur Verstärkung und um die Befestigung zu erleichtern mit einem kreisförmigen Wulst 7, 8 ausgebildet.

In diesem Ausführungsbeispiel ist das Dichtelement 2a einstückig aus einem spritzgießfähigen, hochelastischen Material ausgebildet. Hier wurde ein spezieller additionsvernetzter Silikonkautschuktyp mit einer Härte Shore A von 20, einer Reißdehnung von 800% und einem Weiterreißwiderstand von >20 N/mm verwendet. Dieses Material zeichnet sich auch dadurch aus, dass es gegenüber chemischen Desinfektionsmitteln beständig ist, keine Weichmacher enthält und durch Ultraschall nicht beschädigt werden kann, insbesondere hohen mechanischen Belastungen gewachsen ist.

Das erste Halteteil 4 weist eine als Drehring ausgebildete ringförmige Aufnahmeform 19 auf, die auf ihrer Innenfläche einen ringförmigen Steg 20 besitzt. Auf diesem Steg 20 liegt der eine ringförmige Wulst 7 des Dichtelements 2a die Öffnung 3 umlaufend ununterbrochen auf. Mit einer gelochten Klemmscheibe 21, die mittels eines Gewindes in die Aufnahmeform 19 eingeschraubt ist, wird der Wulst 7 des Dichtelements 2a gegen den Steg 20 des Aufnahmeelements 19 entlang einer geschlossenen, ununterbrochenen Linie angepresst.

In ähnlicher Weise ist das Dichtelement 2a mit dem zweiten Halteteil 5 verbunden. Das zweite Halteteil 5 weist eine als Haltering ausgebildete ringförmige Aufnahmeform 27 mit einem sich an der Innenfläche befindlichen Steg 22 auf. Auf diesem Steg 22 liegt der zweite Wulst 8 des Dichtelements 2a ununterbrochen die Öffnung 3 umlaufend auf. Der zylinderförmige, einseitig offene Behälter 15 ist im Bereich seiner Öffnung flanschartig mit einer ringförmigen Auflagefläche 23 ausgebildet. Über ein Gewinde kann der Haltering 27 auf den flanschartigen Bereich des Behälters 15 aufgeschraubt werden. Im aufgeschraubten Zustand wird der zweite Wulst 8 des Dichtelements 2a zwischen dem Steg 22 des Halterings 27 und dem flanschförmigen Randbereich 23 des Behälters 15 eingeklemmt (der flanschförmige Bereich 23 des Behälters 15 ist somit ein Teil des Halteteils 5).

Erstes Halteteil 4 und zweites Halteteil 5 sind somit mit dem Dichtelement 2a ununterbrochen entlang einer auf der Fläche des Dichtelements 2a die Öffnung 3 des Dichtelements 2a umlaufenden, geschlossenen Linie, nämlich entlang der Wulst 7, 8, befestigt. Aufgrund der Elastizität des Dichtelements 2a und dadurch, dass sich die Klemmwirkung auf das Dichtelement 2a durch das weitere Zuschrauben der Klemmscheibe 21 des ersten Halteteils 4 und das Aufschrauben des Halterings 27 auf den flanschförmigen Randbereich 23 des Behälters 15 weiter verstärken lässt, lässt sich eine sehr hohe Dichtwirkung im Bereich der Wulst 7, 8 des Dichtelements 2a erzeugen, die verhindert, dass an diesen Stellen ein Fluid, insbesondere eine Flüssigkeit oder ein Gas, oder aber auch ein pastöses Medium, hindurchdringen kann.

Erstes Halteteil 4 und zweites Halteteil 5 sind genau um eine Drehachse 9 gegeneinander verdrehbar, wobei die Drehachse 9 der Symmetrieachse 9 des Dichtelements 2a entspricht. Die Auflagenflächen für die Wulste 7, 8 des Dichtelements 2a des Steges des Drehrings 19 des ersten Halteteils 4 und des Stegs 22 des Halterings 27 des zweiten Halteteils 5 sind ebenfalls in genannter Achse rotationssymmetrisch ausgebildet. Die Befestigungsstellen 7, 8 des ersten Halteteils 4 und des zweiten Halteteils 5 an dem Dichtelement 2a liegen somit auf jeweils einem senkrecht zur Symmetrieachse 9 des Dichtelements 2a orientierten ersten bzw. zweiten Kreis. Diese Kreise weisen insbesondere in diesem Ausführungsbeispiel einen identischen Durchmesser auf.

Um die Drehachse 9 festzulegen, weist der Drehring 19 des ersten Halteteils 4 eine in seiner Innenfläche eingebrachte ringförmige Nut 24 auf, in die an der Außenfläche des Halterings 27 des zweiten Halteteils 5 sich befindende ringförmig angeordnete Vorsprünge 25 eingreifen.

Des Weiteren weist das Haltemittel ein Arretiermittel auf, mit dem ersten Halteteil 4 und zweites Halteteil 5 in einer zueinander verdrehten Stellung arretierbar sind.

In diesem Ausführungsbeispiel weist der Haltering 27 des zweiten Halteteils 5 auf der Rückseite des Stegs 22, der Auflagefläche für den Wulst 8 des Dichtelements 2a gegenüberliegend, vereinzelte, um die Drehachse 9 kreisförmige angeordnete Aussparungen 10 auf. Der Drehring 19 des ersten Halteteils 4 weist vereinzelte Vorsprünge 11 auf, die um die Drehachse 9 kreisförmige angeordnet sind, und in die Aussparungen 10 des Halterings 27 eingreifen können. Durch Eingriff der Vorsprünge 11 des Drehrings 19 in die Aussparungen 10 des Halterings 27 wird ein Formschluss gebildet, der erstes und zweites Halteteil 4, 5 in ihrer Position arretiert.

In diesem Ausführungsbeispiel sind die Aussparungen 10 des Halterings 27 auf einer Seite angeschrägt, so dass beim Verdrehen der Halteteile 4, 5 in eine Richtung die Vorsprünge 11 des Drehrings 19 aufgrund der Schräge aus den Aussparungen 10 des zweiten Halteteils 5 gehoben werden. Auf diese Weise ist es möglich, die beiden Halteteile 4, 5 in einer Richtung einfach zu verdrehen, ein Zurückdrehen in die andere Richtung aber blockiert ist. Dies ist insbesondere in diesem Ausführungsbeispiel vorteilhaft, da ein elastisches Dichtelement 2a verwendet wird, welches beim Verdrehen Rückstellkräfte aufbaut.

Das Zurückdrehen der Halteteile 4, 5 kann dadurch bewirkt werden, dass das erste Halteteil 4 leicht angehoben wird, so dass die Vorsprünge 11 des Halteteils 4 aus den Aussparungen 10 des Halteteils 5 gehoben sind. Die Führung der Vorsprünge 25 des zweiten Halteteils 5 in der Nut 24 des ersten Halteteils 4 ist hierfür mit genügend Spiel ausgebildet.

Erstes Halteteil 4 und zweites Halteteil 5 weisen beide eine Öffnung 12, 13 auf, die fluchtend mit der Öffnung 3 des Dichtelements 2a angeordnet sind. Die Öffnung im ersten Halteteil 4 befindet sich in der Klemmscheibe 21, die zweite Öffnung 13 im zweiten Halteteil 5 wird durch die Öffnung einer Lochscheibe 26 gebildet, die knapp unterhalb des Randes im Öffnungsbereich des Behälters 15 in eine sich in der Innenwand des Behälters 15 befindenden Nut eingeklemmt ist. Beide Öffnungen 12, 13 sind zylinderförmig und besitzen die Drehachse 9 der Halteteile 4, 5 bzw. die Symmetrieachse 9 des Dichtelements 2a als Symmetrieachse. Der Durchmesser der Öffnungen 12, 13 ist dabei kleiner als der minimale Durchmesser der Öffnung 3 des Dichtelements 2a, der durch den Scheitel der Wölbung gebildet wird, wobei sich das Dichtelement 2a in einem nicht verdrehten unverformten Zustand, also im Grundzustand, befindet.

Die Verdrehdichtung 1 ist fluiddicht an dem einen offenen Ende des zylinderförmigen Behälters 15 befestigt. Das andere Ende des Behälters ist durch einen Boden abgeschlossen. Im Bereich des Bodens des Behälters 15 ist eine in Form eines Anschlussstutzens ausgebildete Durchführung 16 zum Innenvolumen 18 des Behälters 15 hin vorgesehen, an die beispielsweise über einen Schlauch eine Pumpe angeschlossen werden kann.

Behälter 15, scheibenförmiges Element 21 und Aufnahmeform 19 des ersten Halteteils, sowie Aufnahmeform 21 und scheibenförmiger Einsatz 26 des zweiten Halteteils sind jeweils einstückig aus spritzgießfähigem Kunststoff hergestellt. Speziell für den Behälter 15 wurde ein im Wasser transparentes Material verwendet.

Das Dichtelement 2a wird lediglich an den Wülsten 7, 8 von den Halteteilen gehalten. Der restliche Bereich ist frei und somit verformbar. Durch ein Verdrehen des ersten und zweiten Halteteils 4, 5 verformt sich das von den Halteteilen 4, 5 gehaltene Dichtelement 2a derart, dass sich die Öffnung 3 des Dichtelements 2a in ihrem Öffnungsquerschnitt reduziert. Mit der hier gezeigten Ausführungsform ist sogar ein vollständiges Schließen der Öffnung 3 möglich.

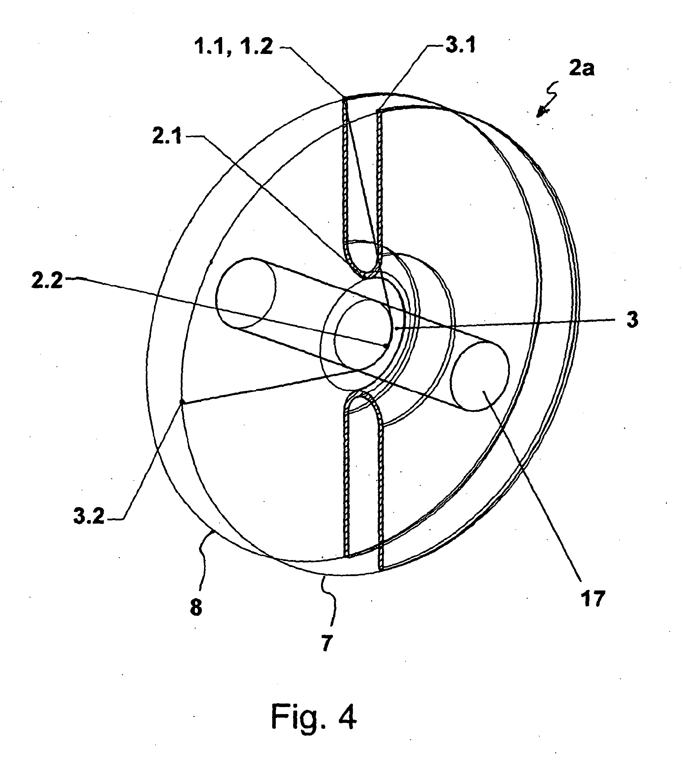

Warum sich eine derartige Verformung des Dichtelements 2a ergibt, soll anhand Fig. 4 näher erläutert werden.

Fig. 4 zeigt das Dichtelement 2a in einer vereinfachten Darstellung. Des Weiteren ist in die Öffnung 3 des Dichtelements 2a ein rohrförmiges Element 17 eingeführt.

Der eine Außenrand 7, gehalten durch das erste Halteteil 4, wird nun gegen den ruhenden Außenrand 8, befestigt am zweiten Halteteil 5, durch Verdrehen der beiden Halteteile gegeneinander verdreht. Es sollen die Verschiebung bestimmter ausgewählter Punkte auf dem Dichtelement 2a von einem Grundzustand 1 in einem verdrehten Zustand 2 betrachtet werden.

Der Punkt 1.1 auf dem unbewegten Außenrand 8 des Dichtelements 2a verändert, da er durch das unbewegte zweite Halteteil 5 festgehalten wird, seine Position während des Verdrehens nicht. Im verdrehten Zustand 2 stimmt somit die Position 1.2 dieses Punktes mit der vorherigen Position 1.1 überein. Der Punkt 3.1, der sich auf dem Außenrand 7 des Dichtelements 2a befindet, wird durch das Drehen des ersten Halteteils 5 in eine Position 3.2 überführt.

Der Punkt 2.1, der sich im Grundzustand des Dichtelements 2a auf der Hälfte der kürzesten auf der Fläche des Dichtelements 2a entlang laufenden Verbindungslinie zwischen den Punkten 1.1 und 3.1 befindet, also auf dem Scheitel der Wölbung des Dichtelements 2a, wird durch die kreisförmige Verschiebung des Punktes 3.1 um das rohrförmige Element 17 herumgeführt, nähert sich diesem spiralförmig an und legt sich schließlich an dessen Oberfläche an, wie es der in Fig. 4 dargestellte Zustand 2.2 zeigt.

Dieses Prinzip gilt für jeden Punkt 1.1'; und 3.1'; die auf dem vom ersten Halteteil 4 bzw. zweiten Halteteil 5 festgehaltenen Außenrand 7 bzw. 8 liegen. Da nur die Außenränder 7, 8 durch die Halteteile gehalten werden, führt dies dazu, dass sich das Dichtelement 2a in der Mitte um das rohrförmige Element 17 zusammenschnürt, insbesondere den im Grundzustand des Dichtelements 2a zwischen rohrförmigem Element 17 und unverformten Dichtelement 2a noch offenen Bereich verschließt. Durch die Stärke der Drehung kann bestimmt werden, wie fest sich das Dichtelement an das rohrförmige Element 17 legt, wodurch sich die Dichtwirkung entsprechend verbessern lässt. Dieses wird unterstützt durch das elastische Material des Dichtelements 2a.

Ohne rohrförmiges Element 17 würde sich das Dichtelement 2a bei genügender Drehung in seinem Zentrum aufwickeln. Auf diese Weise ist ein vollständiges Schließen der Öffnung 3 möglich.

Obige Beschreibung stellt das Prinzip, nach dem sich die Öffnung 3 durch ein Verdrehung der Außenränder 7, 8 des Dichtelements 2a verschließt, sehr vereinfacht dar. In der Realität zieht sich das Dichtelement 2a in seiner Mitte nicht gleichmäßig zusammen, sondern schlägt Falten. Nichtsdestotrotz verkleinert sich der Öffnungsquerschnitt der Öffnung 3, insbesondere ist eine sehr gute Dichtwirkung zwischen Dichtelement 2a und umschlossenem Element 17 möglich. Mit den in Fig. 3a angegebenen Maßen des Dichtelements und dem beschriebenen Material war es insbesondere möglich, für rohrförmige Gegenstände mit einem Durchmesser von 1 bis 10 mm Dichtungen zu erzeugen, die Druckdifferenzen von 0,8 bar problemlos standhielten.

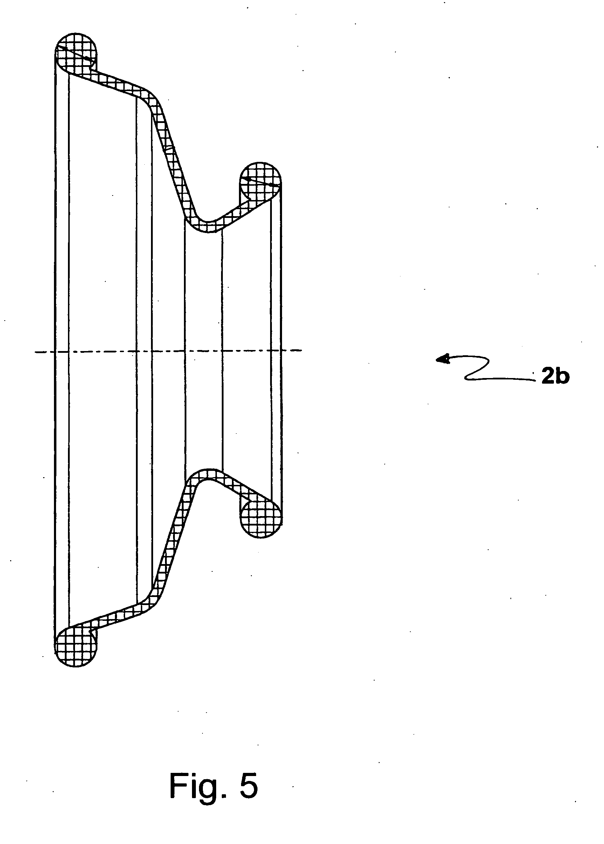

Fig. 5a zeigt eine alternative zweite Ausführungsform eines Dichtelements, welches sich im Rahmen einer erfindungsgemäßen Verdrehdichtung verwenden lässt. Abweichend von der ersten Ausführungsform des Dichtelements 2a besitzen die beiden wulstförmigen Außenränder des ringförmigen, nach innen gewölbten Dichtelements 2b nicht denselben Radius, sondern einer der Außenränder ist mit einem deutlich kleineren Radius ausgebildet. Das Dichtelement 2b ist somit asymmetrisch.

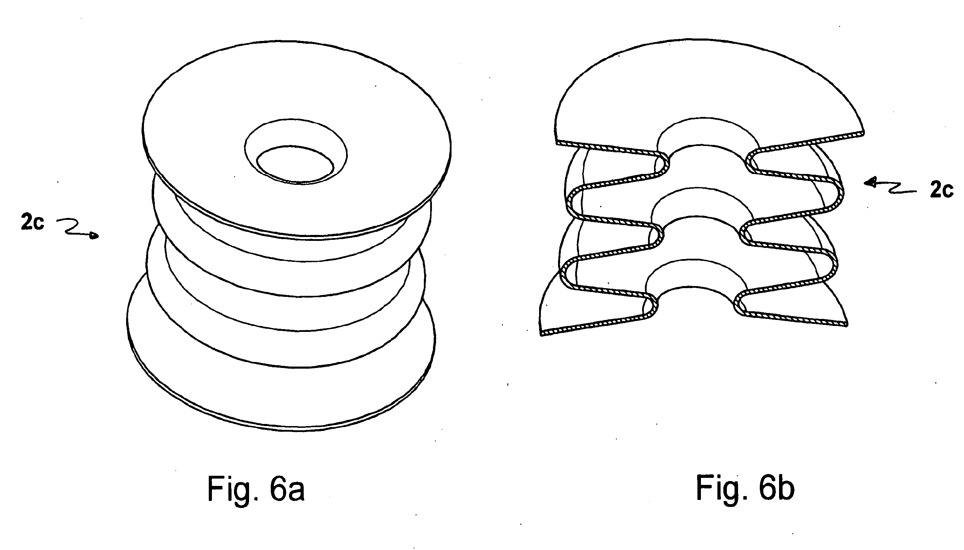

Die Fign. 6a und 6b zeigen eine dritte Ausführungsform eines Dichtelements 2c, welches sich als Teil einer erfindungsgemäßen Verdrehdichtung verwenden lässt. Gemäß dieser dritten Ausführungsform ist das Dichtelement 2c als rotationssymmetrischer Balg mit mehreren Wellungen ausgebildet.

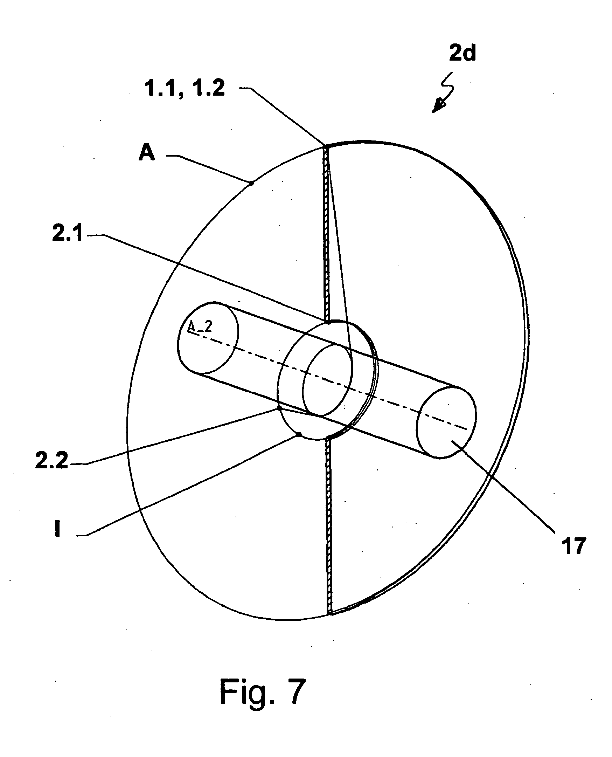

Fig. 7 zeigt eine vierte Ausführungsform eines Dichtelements 2d, welches als Teil einer erfindungsgemäßen Verdrehdichtung 1 verwendet werden kann.

Das Dichtelement 2d ist als Lochscheibe ausgebildet. Durch Drehen des Außenrandes A gegen den Innenrand I verformt sich das Dichtelement 2d derart, dass sich die Öffnung des Dichtelements 2d in ihrem Öffnungsquerschnitt reduziert. In Fig. 7 ist dies analog zur Fig. 4 durch die Position von verschiedenen Punkten im Grundzustand und in einem verdrehten Zustand des Dichtelements 2d vereinfacht dargestellt. In der Realität schlägt sich das Dichtelement 2d auf einer Seite der Scheibe übereinander und schnürt sich dort zusammen, d.h., die ursprüngliche plane Form wird aufgegeben.

Alternativ zu oben beschriebenen Ausführungsformen des Dichtelements ist es grundsätzlich ebenfalls möglich, unelastische Materialien zu verwenden. Auch eine Kombination von starren und elastischen Materialien ist grundsätzlich möglich, insbesondere dann, wenn in einem Teilbereich des Dichtelements Formstabilität gewünscht ist.

Das Gefäß gemäß der beschriebenen Ausführungsform eignet sich insbesondere für die Spülung von hohlen, rohrförmigen Elementen 17, insbesondere von medizinischen Hohlinstrumenten.

Verschiedene Typen von medizinischen Rohrschaft- oder Hohlinstrumenten mit unterschiedlichen Durchmessern können mit einem Ende durch die Öffnungen 12, 3 und 13 der Verdrehdichtung in das Innenvolumen 18 des Behälters 15 eingeführt werden und über ein Verdrehen der Halteteile 4, 5 der Verdrehrichtung 1 gegen den Außenraum sehr gut abgedichtet werden. Dadurch, dass die Öffnungen 12, 13 der Halteteile in nicht verdrehtem Zustand des Dichtelements 2a in ihren Öffnungsquerschnitten kleiner sind als der Öffnungsquerschnitt der Öffnung 3 des Dichtelements 2a wird verhindert, dass beim Einführen des Instruments das Instrument das Dichtelement 2a beschädigt.

Das Instrument kann zusammen mit dem Gefäß 14 in eine Spülflüssigkeit gelegt werden. Durch Pumpen des Innenvolumens 18 über den Anschlussstutzen 16 wird Spülflüssigkeit durch das Hohlinstrument hindurch in das Innenvolumen 18 und dann zur Pumpe gesaugt.

Aufgrund der guten Dichtwirkung der Verdrehdichtung kann gewährleistet werden, dass die Spülflüssigkeit tatsächlich durch das Hohlinstrument gesaugt wird und damit ein Reinigungseffekt eintritt. Zusätzlich ist es möglich, die Reinigungsflüssigkeit mit Ultraschall zu beaufschlagen, wobei das Gefäß 14 den Ultraschall, vorausgesetzt, dass das Innenvolumen 18 mit Flüssigkeit gefüllt ist, auch an dem im Gefäß 14 liegenden Bereich des Hohlinstruments weiterleiten kann.

## Patentansprüche

1. Einrichtung zur Spülung von hohlen, rohrförmigen Elementen (17), insbesondere von medizinischen Hohlinstrumenten, enthaltend ein Gefäß (14), **dadurch gekennzeichnet dass** das Gefäß (14) enthaltend
einen Deckel und einen Behälter (15), bei dem der Deckel durch eine Verdrehdichtung (1) gebildet wird, wobei ein Halteteil (5) der Verdrehdichtung (1) fluiddicht an dem Behälter (15) befestigt ist, und wobei das Gefäß (14) einen zum Innenvolumen (18) des Behälters (15) hin durchgeführten Anschluss (16) für eine Pumpe zum Pumpen des Innenvolumens (18) des Behälters (15) aufweist, wobei
die Verdrehdichtung (1) ein Dichtelement (2a) und ein Haltemittel enthält, wobei das Dichtelement (2a) als flächiges Gebilde mit einer in ihrem Öffnungsquerschnitt veränderbaren Öffnung (3) ausgebildet ist und das Haltemittel ein erstes Halteteil (4) und ein zweites Halteteil (5) aufweist, die gegeneinander verdrehbar sind, und erstes Halteteil (4) und zweites Halteteil (5) jeweils zumindest an einer Befestigungsstelle (7, 8) mit dem Dichtelement (2a) fest verbunden sind, wobei die Befestigungsstellen (7, 8) derart gewählt sind, dass durch ein Verdrehen des ersten (4) und zweiten Halteteils (5) das Dichtelement (2a) derart verformbar ist, dass sich die Öffnung (3) des Dichtelementes (2) in ihrem Öffnungsquerschnitt reduziert.

2. Einrichtung nach Anspruch 1, **dadurch gekennzeichnet, dass** zumindest erstes (4) und/oder zweites Halteteil (5) mit dem Dichtelement (2a) ununterbrochen entlang einer auf der Fläche des Dichtelementes (2a) die Öffnung (3) des Dichtelementes (2a) umlaufenden, geschlossenen Linie (7, 8) befestigt ist.

3. Einrichtung nach einem der vorhergehenden Ansprüche, **dadurch gekennzeichnet, dass** das erste Halteteil (4) gegen das zweite Halteteil (5) um eine Drehachse (9) verdrehbar ist, wobei die Befestigungsstellen (7, 8) von erstem und zweitem Halteteil (4, 5) an dem Dichtelement (2a) derart gewählt sind, dass es keine senkrecht zur Drehachse (9) orientierte Ebene gibt, auf der sich sowohl eine Befestigungsstelle (7) des ersten (4) als auch eine Befestigungsstelle (8) des zweiten Halteteils (5) befinden.

4. Einrichtung nach einem der vorhergehenden Ansprüche, **dadurch gekennzeichnet, dass** das Dichtelement in Form einer Scheibe (2d), eines Ringes (2a, 2b) oder eines Schlauches (2a, 2c) ausgebildet ist.

5. Einrichtung nach einem der vorhergehenden Ansprüchen, **dadurch gekennzeichnet, dass** das Dichtelement (2a) zumindest in einem Teilbereich umlaufend der Öffnung (3) nach innen gewölbt ist, und sich die Befestigungsstellen (7) des ersten Halteteils (4) in dem einen und die Befestigungsstellen (8) des zweiten Halteteils (5) in dem anderen der durch den Scheitel der Wölbung voneinander getrennten Bereiche des Dichtelementes (2a) befinden.

6. Einrichtung nach einem der vorhergehenden Ansprüche, **dadurch gekennzeichnet, dass** das Dichtelement (2a) rotationssymmetrisch ausgebildet ist, und die Befestigungsstellen (7, 8) des ersten Halteteils (4) und des zweiten Halteteils (5) an dem Dichtelement (2a) auf jeweils einem senkrecht zur Symmetrieachse (9) des Dichtelementes (2) orientierten ersten bzw. zweiten Kreis liegen.

7. Einrichtung nach Anspruch 6, **dadurch gekennzeichnet, dass** der Radius des ersten Kreises (7) mit dem des zweiten Kreises (8) identisch ist.

8. Einrichtung nach einem der vorhergehenden Ansprüche, **dadurch gekennzeichnet, dass** das erste Halteteil (4) gegen das zweite Halteteil (5) um genau eine Drehachse (9) verdrehbar ist.

9. Einrichtung nach einem der vorhergehenden Ansprüche, **dadurch gekennzeichnet, dass** das Haltemittel ein Arretiermittel (10, 11) aufweist, mit dem erstes Halteteil (4) und zweites Halteteil (5) in einer zueinander verdrehten Stellung arretierbar sind.

10. Einrichtung nach einem der vorhergehenden Ansprüche, **dadurch gekennzeichnet, dass** das erste (4) und/oder das zweite Halteteil (5) eine Öffnung (12, 13) aufweist bzw. aufweisen, die fluchtend mit der Öffnung (3) des Dichtelements (2a) angeordnet ist bzw. sind.

11. Einrichtung nach Anspruch 10, **dadurch gekennzeichnet, dass** die Öffnungsquerschnitte der Öffnungen (12, 13) des ersten (4) und zweiten Halteteils (5) in einem Grundzustand des Dichtelementes (2a) in Fluchtlinie (9) vollständig den Öffnungsquerschnitt des Dichtelementes (2a) überdecken.

12. Einrichtung nach einem der vorhergehenden Ansprüchen, **dadurch gekennzeichnet, dass** das Dichtelement (2a) zumindest bereichsweise, vorzugsweise vollständig, aus einem elastischen Material besteht.

13. Einrichtung nach Anspruch 12, **dadurch gekennzeichnet, dass** das Dichtelement (2a) zumindest bereichsweise, vorzugsweise vollständig, aus Silikonkautschuk, thermoplastischen Elastomeren, Naturkautschuk und/oder Polymerlatex besteht.

14. Einrichtung nach einem der Ansprüche 12 oder 13, **dadurch gekennzeichnet, dass** der elastische Bereich des Dichtelementes (2) eine Reißdehnung von mindestens 300%, vorzugsweise mindestens 600%, besonders bevorzugt mindestens 800% aufweist.

## Claims

1. A device for rinsing hollow, tubular elements (17), in particular medical hollow instruments, containing a vessel (14), **characterized in that** the vessel (14) contain[s] a cover and a container (15), in which the cover is formed by a torsion seal (1), wherein a retaining part (5) of the torsion seal (1) is fastened to the container (15) in a fluid-tight manner, and wherein the vessel (14) has an attachment (16) passing through to the internal volume (18) of the container (15) for a pump for pumping the internal volume (18) of the container (15), wherein the torsion seal (1) contains a sealing element (2a) and a retaining means, wherein the sealing element (2a) is constructed in the form of a planar structure with an opening (3) capable of being varied in its opening cross-section, and the retaining means has a first retaining part (4) and a second retaining part (5) which are capable of being turned with respect to each other, and the first retaining part (4) and the second retaining part (5) are joined to the sealing element (2a) in a fixed manner at least at one fastening point (7, 8) in each case, wherein the fastening points (7, 8) are selected in such a way that the sealing element (2a) is capable of being deformed by a turning of the first retaining part (4) and the second retaining part (5) in such a way that the opening (3) of the sealing element (2) is reduced in cross-section.

2. A device according to Claim 1, **characterized in that** at least the first retaining part (4) and/or the second retaining part (5) is or are fastened to the sealing element (2a) in an uninterrupted manner along a continuous line (7, 8) running around the opening (3) of the sealing element (2a) on the face of the sealing element (2a).

3. A device according to one of the preceding Claims, **characterized in that** the first retaining part (4) is capable of being turned with respect to the second retaining part (5) about an axis of rotation (9), wherein the fastening points (7, 8) of the first retaining part (4) and the second retaining part (5) on the sealing element (2a) are selected in such a way that there is no plane which is orientated at a right angle to the axis of rotation (9) and on which both a fastening point (7) of the first retaining part (4) and a fastening point (8) of the second retaining part (5) are present.

4. A device according to any one of the preceding Claims, **characterized in that** the sealing element is constructed in the form of a disc (2d), a ring (2a, 2b) or a hose (2a, 2c).

5. A device according to any one of the preceding Claims, **characterized in that** the sealing element (2a) is curved inwards at least in a partial region running around the opening (3), and the fastening points (7) of the first retaining part (4) are present in one of the regions of the sealing element (2a) and the fastening points (8) the second retaining part (5) are present in the other of the regions of the sealing element (2a) which are separated from each other by the apex of the curvature.

6. A device according to any one of the preceding Claims, **characterized in that** the sealing element (2a) is made rotationally symmetrical, and the fastening points (7, 8) of the first retaining part (4) and the second retaining part (5) on the sealing element (2a) are situated on one first and second circle respectively orientated at a right angle to the axis of symmetry (9) of the sealing element (2) in each case.

7. A device according to Claim 6, **characterized in that** the radius of the first circle (7) is identical with that of the second circle (8).

8. A device according to any one of the preceding Claims, **characterized in that** the first retaining part (4) is capable of being turned with respect to the second retaining part (5) about precisely one axis of symmetry (9).

9. A device according to any one of the preceding Claims, **characterized in that** the retaining means has a locking means (10, 11) by which the first retaining part (4) and the second retaining part (5) are capable of being locked in a position turned with respect to each other.

10. A device according to any one of the preceding Claims, **characterized in that** the first retaining part (4) and/or the second retaining part (5) has or have an opening (12, 13) which is or are arranged in alignment with the opening (3) of the sealing element (2a).

11. A device according to Claim 10, **characterized in that** the opening cross-sections of the openings (12, 13) of the first retaining part (4) and the second retaining part (5) completely overlap the opening cross-section of the sealing element (2a) in a normal position of the sealing element (2a).

12. A device according to any one of the preceding Claims, **characterized in that** the sealing element (2a) consists at least locally, and preferably completely, of a resilient material.

13. A device according to Claim 12, **characterized in that** the sealing element (2a) consists at least locally, and preferably completely, of silicone rubber, thermoplastic elastomers, natural rubber and/or polymer dispersion.

14. A device according to one of Claims 12 or [*sic*] 13, **characterized in that** the resilient range of the sealing element (2) has an elongation at break of at least 300 %, preferably of at least 600 %, and, in a particularly preferred manner, of at least 800 %.

## Revendications

1. Dispositif pour le rinçage d'éléments creux tubulaires (17), en particulier celui d'instruments médicaux creux, comprenant un récipient (14),
**caractérisé en ce que** le récipient (14) comprend :
un couvercle et une cuve (15), où le couvercle est constitué par un joint d'étanchéité à torsion (1), un élément de maintien (5) du joint d'étanchéité à torsion (1) étant fixé sur la cuve (15) de manière étanche aux fluides, et le récipient (14) comportant un raccord (16) débouchant dans le volume intérieur (18) de la cuve (15), pour le raccordement d'une pompe destinée à pomper le volume intérieur (18) de la cuve (15),
le joint d'étanchéité à torsion (1) comprenant un élément d'étanchéité (2a) et un moyen de maintien, l'élément d'étanchéité (2a) étant réalisé sous forme d'un corps plan ayant une ouverture (3) dont la section d'ouverture est variable, et le moyen de maintien comprenant une première pièce de maintien (4) et une deuxième pièce de maintien (5), pouvant être tournées l'une par rapport à l'autre, et la première pièce de maintien (4) et la deuxième pièce de maintien (5) étant reliées de façon fixe à l'élément d'étanchéité (2a) par au moins un endroit de fixation respectif (7, 8), les endroits de fixation (7, 8) étant choisis de telle façon qu'en effectuant une rotation relative entre les première (4) et deuxième (5) pièces de maintien, l'élément d'étanchéité puisse être déformé de telle sorte que la section d'ouverture de l'ouverture (3) de l'élément d'étanchéité (2) se réduise.

2. Dispositif selon la revendication 1, **caractérisé en ce que**
au moins la première (4) et/ou la deuxième (5) pièce(s) de maintien est/sont reliée(s) à l'élément d'étanchéité (2a) de façon ininterrompue le long d'une ligne fermée (7, 8) s'étendant à la surface de l'élément d'étanchéité (2a), entourant l'ouverture (3) de l'élément d'étanchéité (2a).

3. Dispositif selon l'une des revendications précédentes, **caractérisé en ce que**
la première pièce de maintien (4) peut être entraînée en rotation par rapport à la deuxième pièce de maintien (5) autour d'un axe de rotation (9), les endroits de fixation (7, 8) des première et deuxième pièces de maintien (4, 5) sur l'élément d'étanchéité étant choisis de telle façon qu'aucun plan orienté perpendiculairement à l'axe de rotation (9) n'existe, dans lequel se trouveraient à la fois un endroit de fixation (7) de la première pièce (4), et un endroit de fixation (8) de la deuxième pièce de maintien (5).

4. Dispositif selon l'une des revendications précédentes, **caractérisé en ce que** l'élément d'étanchéité est réalisé sous forme d'une rondelle (2d), d'un anneau (2a, 2b), ou d'un tube flexible (2a, 2c).

5. Dispositif selon l'une des revendications précédentes, **caractérisé en ce que**
l'élément d'étanchéité (2a) est bombé vers l'intérieur au moins sur une partie de celui-ci, en suivant la périphérie de l'ouverture (3), et que les endroits de fixation (7) de la première pièce de maintien (4) se situent dans l'une, et les endroits de fixation (8) de la deuxième pièce de maintien (5) se situent dans l'autre des zones de l'élément d'étanchéité (2a), qui sont séparées l'une de l'autre par le sommet du bombé.

6. Dispositif selon l'une des revendications précédentes, **caractérisé en ce que**
l'élément d'étanchéité (2a) est formé de façon symétrique en rotation, et que les endroits de fixation (7, 8) de la première pièce de maintien (4) et de la deuxième pièce de maintien (5) sont situés sur l'élément d'étanchéité (2a), sur un premier, voire sur un deuxième cercle, respectivement orienté perpendiculairement à l'axe de symétrie (9) de l'élément d'étanchéité (2).

7. Dispositif selon la revendication 6, **caractérisé en ce que**
le rayon du premier cercle (7) est identique à celui du deuxième cercle (8).

8. Dispositif selon l'une des revendications précédentes, **caractérisé en ce que**
la première pièce de maintien (4) peut être entraînée en rotation par rapport à la deuxième pièce de maintien (5) exactement autour d'un axe de rotation (9).

9. Dispositif selon l'une des revendications précédentes, **caractérisé en ce que** le moyen de maintien comprend un moyen d'arrêt (10, 11) à l'aide duquel les première pièce de maintien (4) et deuxième pièce de maintient (5) peuvent être arrêtées dans une position de rotation relative, l'une par rapport à l'autre.

10. Dispositif selon l'une des revendications précédentes, **caractérisé en ce que** la première (4) et/ou la deuxième (5) pièce(s) de maintien présente(nt) une ouverture (12, 13), ouverture(s) qui est (sont) disposée(s) en alignement avec l'ouverture (3) de l'élément d'étanchéité (2a).

11. Dispositif selon la revendication 10, **caractérisé en ce que**
les sections d'ouverture des ouvertures (12, 13) des première (4) et deuxième (5) pièces de maintien recouvrent entièrement, dans un état de base de l'élément d'étanchéité (2a), la section d'ouverture de l'élément d'étanchéité (2a), dans l'axe d'alignement (9).

12. Dispositif selon l'une des revendications précédentes, **caractérisé en ce que**
l'élément d'étanchéité (2a) est constitué au moins en partie, de préférence entièrement, d'un matériau élastique.

13. Dispositif selon la revendication 12, **caractérisé en ce que**
l'élément d'étanchéité (2a) est constitué au moins en partie, de préférence entièrement, de caoutchouc au silicone, d'élastomères thermoplastiques, de caoutchouc naturel et/ou de latex polymérisé.

14. Dispositif selon l'une des revendications 12 ou 13, **caractérisé en ce que**
la zone élastique de l'élément d'étanchéité (2) présente une élongation à la rupture d'au moins 300 %, de préférence d'au moins 600 %, et de façon particulièrement préférée d'au moins 800 %.
